# FASCICULE DE BREVET EUROPEEN

(11) **EP 1 333 803 B1**
(45) Date de publication et mention de la délivrance du brevet: **25.04.2007**
(21) Numéro de dépôt: 01993445.4
(22) Date de dépôt: 13.11.2001
(51) Int. Cl.: A61Q 17/00, A61K 8/60

(54) **UTILISATION DE CARBOHYDRATE POUR AMELIORER LA FONCTION BARRIERE DE LA PEAU**
VERWENDUNG VON KOHLENHYDRATEN ZUR VERBESSERUNG DER HAUT BARRIEREFUNKTION
USE OF CARBOHYDRATE FOR ENHANCING THE SKIN BARRIER FUNCTION

(30) Priorité: 13.11.2000 FR 0014557
(43) Date de publication de la demande: 13.08.2003
(73) Titulaire: L'ORÉAL, 75008 Paris (FR)
(72) Inventeur: MEHUL, Bruno, F-92800 Villejuif (FR)
(74) Mandataire: Desaix, Anne
(86) Numéro de dépôt international: PCT/FR2001/003550
(87) Numéro de publication internationale: WO 2002/038110

(56) Documents cités:
- WO-A-94/12651
- WO-A-97/12597
- FR-A- 2 696 467
- US-A- 5 507 970
- DATABASE WPI Week 198641 Derwent Publications Ltd., London, GB; AN 1986-267846 XP002177221 & JP 61 194007 A (DAIICHI KOGYO & SHISEIDO), 28 août 1986 (1986-08-28)
- DATABASE WPI Week 198843 Derwent Publications Ltd., London, GB; AN 1988-303604 XP002177222 & JP 63 222698 A (ASAHI DENKA), 16 septembre 1988 (1988-09-16)

## Description

L'invention a trait à l'utilisation de dérivés carbohydrates dans une composition, pour améliorer la fonction barrière de la peau. Elle porte aussi sur un procédé de traitement cosmétique destiné à améliorer la fonction barrière de la peau par l'application sur la peau de compositions comprenant des dérivés carbohydrates.

La peau humaine est constituée de deux compartiments à savoir un compartiment profond, le derme et un compartiment superficiel, l'épiderme.

L'épiderme est en contact avec l'environnement extérieur. Son rôle consiste à protéger l'organisme de la déshydratation et des agressions extérieures, chimiques, mécaniques ou infectieuses.

Les cellules constituant l'épiderme sont délimitées par une structure lipidique intercellulaire. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides. Ces lipides sont organisés en structures lamellaires spécifiques dont l'intégrité dépend non seulement de la qualité des fractions présentes mais aussi de leur proportion respective. Cette structure lamellaire des lipides est responsable de la souplesse de la peau. Parmi les lipides, les sphingolipides (céramides) sont essentiels pour le maintien de la structure multilamellaire des lipides intercoméocytaires. Ils sont essentiels pour les échanges en eau et la fonction « barrière » de l'épiderme.

Les lipides intercoméocytaires subissent des modifications. Cette maturation est nécessaire à l'établissement d'une bonne fonction barrière. La déglycosilation de précurseurs lipidiques comme le glucosylcéramide en céramide est modulé par l'action de glycosidases (glucosidase) endogènes spécifiques. Cette déglycosilation est par conséquent une étape importante dans la mise en place de la fonction barrière de la peau.

Les lipides de la peau, particulièrement de l'épiderme, sont influencés par des facteurs génétiques, le vieillissement, les régimes alimentaires, les facteurs environnementaux, les agressions et/ou certaines pathologies (scorbut ou pellagre par exemple). Ces facteurs ont pour conséquence d'altérer ou encore de modifier la composition des lipides de la peau ou d'en diminuer la quantité, conduisant à une peau sèche.

L'invention résulte d'études *in vitro* et *in vivo* de l'effet de dérivés de carbohydrates sur la peau.

Une utilisation de ces dérivés de carbohydrates pour favoriser la desquamation de la peau a déjà été décrite dans la demande de brevet « Utilisation de carbohydrate pour favoriser la desquamation de la peau » (WO-A-97/12597 L'Oréal).

FR 2 696 467 concerne un procédé de préparation de monoesters d'acides gras du D-fructose et leur utilisation dans les domaines cosmétiques buccodentaire, pharmaceutique et alimentaire.

De manière surprenante, il a été maintenant montré un nouvel effet des dérivés de carbohydrates, obtenu par la stimulation spécifique de certaines β-glucosidases se manifestant par l'amélioration de la fonction barrière de la peau et/ou des muqueuses, particulièrement lorsque les dérivés de carbohydrates sont appliqués par voie topique.

En d'autres termes, l'invention a pour objet l'utilisation de compositions utilisées pour améliorer la fonction barrière de la peau, cette fonction étant corrélable à l'activité de β-glucosidases, de sorte que l'amélioration de la fonction barrière peut être révélée par une stimulation de l'activité de β-glucosidases.

Les β-glucosidases entrent dans le catabolisme des glycolipides, en particulier des glucosyl-céramides. Une augmentation spécifique de l'activité β-glucosidase permet ainsi d'augmenter la teneur en céramides des lipides de la peau, améliorant par là, la fonction barrière de la peau.

Il a été montré en particulier un effet de l'O-octanoyl-6'-maltose sur la stimulation de l'activité de certaines glycosidases et plus particulièrement de la β-D-glucosidase du stratum corneum. Cet effet est par ailleurs corrélé *in vivo* à une augmentation sensible de la fonction barrière de la peau.

L'invention a donc pour objet l'utilisation, dans une composition pour améliorer la fonction barrière de la peau d'octanoyl-6'-maltose, et éventuellement, d'au moins un carbohydrate ou dérivé de carbohydrate répondant à la formule générale (I),

R-X-A (I)

dans laquelle A représente une chaîne composée de une à vingt unités carbohydrate ou dérivé de carbohydrate, comprenant chacune 3 à 6 atomes de carbone, reliées entre elles, de préférence par des ponts acétals, chacune de ces unités pouvant être éventuellement substituée, par exemple par un halogène, par une fonction amine, une fonction acide, une fonction ester, un thiol, une fonction alcoxy, une fonction thio-éther, une fonction thio-ester, une fonction amide, une fonction carbamate, une fonction urée,
R représente une chaîne alkyle ou une chaîne alcényle, comprenant de 1 à 24, préférentiellement de 4 à 24, atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate, une fonction urée,
X représente une fonction reliant R et A, comme par exemple une fonction amine, éther, amide, ester, urée, carbamate, thioester, thioéther, sulfonamide,

De façon préférentielle, R représente une chaîne alkyle ou une chaîne alcényle, comprenant de 4 à 24 atomes de carbone, ramifiée ou linéaire, portant éventuellement une fonction hydroxyle.

Chacune des unités carbohydrate composant A peut être un sucre ou un dérivé de sucre. Par exemple, chaque unité composant A peut être un sucre réduit, un sucre aminé ou un sucre porteur d'une fonction acide carboxylique.

Parmi les sucres, ou les dérivés de sucre, pouvant entrer dans la constitution de A, on citera par exemple les produits suivants, qui sont disponibles commercialement, éventuellement sous forme de sel : la N-acétyl-D-galactosamine, la N-acétyl-D-glucosamine, l'acide N-acétyl-neuraminique, l'adonitol, le β-D-allose, le β-D-altrose, le 6-amino -6-déoxy-D-glucose, le 1,6-anhydroglucose, l'acide arabinique, l'arabinogalactan, le D-arabinose, le L-arabinose, le D,L-arabinose, le D-arabitol, le D-cellobiose, la D-glucosamine, la D-galactosamine, le 2-déoxy-D-glucose, le 6-déoxy-D-galactose, le 6-déoxy-L-galactose, le galactitol, le mésoérythritol, le D- érythrose, le D-fructose, le D-fucose, le L-fucose, l'acide D-galactarique, le galactitol, le galactomannane, le D-galactono-1,4-lactone, le L-galactono-1,4 -lactone, la D-galactosamine, le D-galactose, le L-galactose, l'acide D-galacturonique, le β-gentiobiose, la glucamine, l'acide D-glucarique, le D-glucono-1,5-lactone, le L-glucono-1,5-lactone, la D-glucosamine, l'acide D-glucosaminique, l'acide D-glucoronique, le L-glucose, le D-glucose, l'isomaltitol, l'isomaltotriose, l'isomaltose, l'acide lactobionique, le D-lactose, le lactulose, le D-lyxose, le L-lyxose, la lyxosamine, le maltitol, le D-maltose, le maltotétraose, le maltotriitol, le maltotriose, la D-mannosamine, le D-mannose, le L-mannose, le D-mélézitose, le D-mélibiose, le D-raffinose, l'undeca-acétate de D-raffinose, le L-rhamnose, le D-ribose, le L-ribose, le D-ribulose, le rutinose, le D-saccharose, l'α-sophorose, le sorbitol, le D-tagatose, le D-talose, le D-thréose, le turanose, le D-xylitol, le D-xylose, le L-xylose, le D, L-xylose.

De façon préférentielle A sera choisie parmi les chaînes hydrocarbonées suivantes :
la D-glucosamine ou 2-amino-2 déoxy-D-glucose, la D-glucamine ou 1-amino-1-déoxy-D-glucitol, la N-méthyl-glucamine, le D-glucose, le D-maltose, le sorbitol, le maltitol.

De façon préférentielle R comprend 4 à 16 atomes de carbone comme par exemple les radicaux n-butyle, n-octyle, 2-éthyl-hexyle, n-dodécyle.

Selon l'invention, les compositions préférées comprendront au moins un produit choisi parmi :
le N-butanoyl-D-glucosamine, le N-octanoyl-D-glucosamine, le N-octyloxycarbonyl-N-méthyl-D-glucamine, le N-2-éthyl-hexyloxycarbonyl-N-méthyl-D-glucamine, le 6'-0-octanoyl-D-maltose, le 6'-0-dodécanoyl-D-maltose.

La préparation de produits (I) est bien connue de l'homme du métier, on pourra par exemple se référer aux documents suivants : FR-A-2703993, FR-A-2715933, EP-A-577506, EP-A-566438 et EP-A-485251.

Un dérivé de carbohydrate répondant à la formule (I) est le O-octanoyl-6'-maltose.

Dans les compositions l'octanoyl-6'-maltose ou son mélange avec le(s) carbohydrate(s) selon (I) peut(vent) être utilisé(s) en une quantité allant de 0,05 à 20 % en poids par rapport au poids total de la composition et en particulier en une quantité allant de 0,2 à 10 % et mieux de 0,5 à 5 % en poids par rapport au poids total de la composition.

Les carbohydrates selon (I) peuvent être sélectionnés à l'aide d'un test *in vitro*, décrit dans la partie expérimentale (partie I.1).

Par conséquent, l'invention porte sur l'utilisation de compositions telles que décrites précédemment, comprenant un dérivé de carbohydrate sélectionné à l'aide d'un test *in vitro* permettant de quantifier un effet stimulateur dudit dérivé sur l'activité β-D-glucosidase, ledit test comprenant les étapes suivantes :
a) la réalisation d'un mélange constitué du dérivé de carbohydrate, d'une β-D-glucosidase, d'un substrat chromogénique de ladite β-D-glucosidase, et d'une solution tampon appropriée ;
b) la quantification de la vitesse de la réaction enzymatique de la β-glucosidase, en particulier par le dosage de la quantité de chromophores libérés par clivage du substrat chromogénique ; et,
c) la sélection de dérivés de carbohydrate avec lesquels la vitesse de la réaction est augmentée par rapport à la vitesse de la réaction mesurée dans une solution contrôle en absence de dérivés.

Les dérivés de carbohydrates sont utilisés selon l'invention dans une composition contenant un milieu cosmétiquement ou dermatologiquement acceptable, c'est-à-dire un milieu compatible avec la peau, les ongles, les muqueuses, les tissus et les cheveux. Selon un mode préféré de réalisation de l'invention, la composition a un pH proche de celui de la peau, compris entre 4 et 7. Lorsqu'elle est appliquée par voie topique, la composition comprenant un ou plusieurs dérivés de carbohydrates peut être appliquée sur le visage, le cou, les cheveux, les muqueuses et les ongles ou tout autre zone cutanée du corps.

La composition se présente de préférence sous une forme appropriée pour une administration par voie topique. Elle se présente notamment sous forme de solutions hydroalcooliques ou huileuses, de dispersions du type lotion ou sérum, de gels anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de micro-émulsions, ou encore de micro-capsules, de micro-particules, ou de dispersions vésiculaires de type ionique et/ou non ionique. Ces compositions sont préparées selon les méthodes usuelles.

Les compositions peuvent être également utilisées pour les cheveux sous forme de solutions alcooliques ou hydroalcooliques, ou sous forme de crèmes, de gels, d'émulsions, de mousses.

Les quantités des différents constituants des compositions utilisées selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

De façon connue, les compositions utilisées selon l'invention peuvent contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges et les matières colorantes. Les compositions utilisées selon l'invention peuvent en outre contenir tout autre composé ayant des propriétés hydratantes et/ou améliorant la fonction barrière. Les quantités de ces différents adjuvants sont celles classiquement utilisées dans les domaines considérés, et par exemple de 0,01% à 20% du poids total de la composition. Bien entendu, l'homme de l'art veillera à choisir ce ou ces éventuels additifs et/ou leurs quantités de manière telle que les propriétés avantageuses attachées intrinsèquement à la composition conforme à l'invention, ne soient pas, ou substantiellement pas, altérées par la ou les adjonctions envisagées.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émultionnants utilisables dans l'invention, on peut citer le Polysorbate 60 et le stéarage de sorbitane vendus respectivement sous les dénominations commerciales Tween 60 et Span 60 par la Société ICI. On peut y ajouter des co-émulsionnants tels que le PPG-3 myristyl éther vendu sous la dénomination commerciale Emcol 249-3K par la société Witco.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sols métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

En vue de lutter efficacement contre le photovieillissement, il est en outre possible d'ajouter à la composition utilisée selon l'invention un ou plusieurs filtres solaires complémentaires, actifs dans l'UVA et/ou l'UVB, hydrophiles ou lipophiles, comportant éventuellement une fonction sulfonique. Le filtre solaire est de préférence choisi parmi les fitres organiques et/ou les filtres minéraux.

Comme filtres organiques, on peut notamment citer les dérivés cinnamiques, les dérivés salicyliques, les dérivés du camphre, les dérivés de triazine, les dérivés de la benzophénone, les dérivés du dibenzoylméthane, les dérivés de β- β-diphénylacrylate, les dérivés de l'acide p-aminobenzoïque, les polymères filtres et silicones filtres décrits dans la demande WO-93/04665 ou encore les filtres organiques décrits dans la demande de brevet EP-A 0 487 404.

Comme filtres minéraux, on peut notamment citer des pigments ou bien encore des nanopigments (taille moyenne des particules primaires : généralement entre 5 nm et 100 nm, de préférence entre 10 et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0 518 772 et EP-A-0 518 773.

Comme exemples de filtre solaires complémentaires actifs dans l'UV-A et/ou l'UV-B, on peut citer :
- l'acide p-aminobenzoïque,
- le p-aminobenzoate oxyéthyléné (25 mol),
- le p-diméthylaminobenzoate de 2-éthylhexyle,
- le p-aminobenzoate d'éthyle N-oxypropyléné,
- le p-aminobenzoate de glycérol,
- le salicylate d'homomenthyle,
- le salicylate de 2-éthylhexyle,
- le salicylate de triéthanolamine,
- le salicylate de 4-isopropylbenzyle,
- le 4-ter-butyl-4'-méthoxy-dibenzoylméthane (PARSOL 1789 de GIVAUDAN ROURE)
- le p-méthoxycinnamate de 2-éthylhexyle (PARSOL MCX de GIVAUDAN ROURE)
- le 4-isopropyl-dibenzoylméthane (EUSOLEX 8020 de MERCK),
- l'anthranilate de menthyle,
- le 2-éthylhexyl-2-cyano-3,3'-diphénylacrylate, (UVINUL N539 de BASF),
- l'éthyl-2-cyano-3,3'-diphénylacrylate,
- l'acide 2-phényl benzimidazole 5-sulfonique et ses sels,
- le 3-(4'-triméthylammonium)-benzylidèn-boman-2-on-méthylsulfate,
- le 2-hydroxy-4-méthoxybenzophénone (UVINUL MS 40 de BASF),
- le 2-hydroxy-4-méthoxybenzophénone-5-sulfonate (UVINUL MS 40 de BASF),
- le 2,4-dihydroxybenzophénone (UVINUL 400 de BASF),
- le 2,2',4,4'-tétrahydroxybenzophénone (UVINUL D 50 de BASF),
- le 2,2'-dihydroxy-4,4'-diméthoxybenzophénone (HELISORB II de NORQUAY),
- le 2-hydroxy-4-n-octoxybenzophénone,
- le 2-hydroxy-4-méthoxy-4'-méthylbenzophénone,
- l'acide α-(2-oxobom-3-ylidène)-tolyl-4-sulfonique et ses sels,
- le 3-(4'-sulfo)benzylidèn-boman-2-one et ses sels,
- le 3-(4'-méthylbenzylidène)-d,l-camphre,
- le 3-benzylidène-d,l-camphre,
- l'acide benzène 1,4-di(3-méthylidène-10-camphosulfonique) et ses sels (MEXORYL SX de CHIMEX),
- l'acide urocanique,
- le 2,4-6-tris-[p-(2'-éthylhexyl-1'-oxycarbonyl)anilino]-1,3,5-triazine,
- le 2-[p-(tertiobutylamido)anilino]-4,6-bis [p-(2'-éthylhexyl-1'-oxycarbonyl)anilino]-1,3,5-triazine,
- le 2,4-bis{[4-2-éthyl-hexyloxyl]-2-hydroxyl-phenyl}-6-(4-méthoxyphenyl)-1,3,5-triazine,
- le polymère de N-(2 et 4)-[2-oxobom-3-ylidèn)méthyl)benzyl]-acrylamide,
- l'acide 4,4-bis-benzimidazolyl-phénylèn-3,3',5,5'-tétrasulfonique et ses sels,
- le 2,2'-méthylèn-bis-[6-(2H-benzotriazol-2-yl)-4-(1,1,3,3-tétraméthylbutyl) phénol],
- les polyorganosiloxanes à fonction malonate.

L'invention porte aussi sur un procédé de traitement cosmétique, par l'application des compositions telles que définies ci-dessus, selon la technique d'utilisation habituelle de ces compositions, ledit procédé cosmétique permettant l'amélioration de la fonction barrière de la peau. Par exemple : application de crèmes, de gels, de sérums, de pommades, de lotions, de laits sur la peau, le cuir chevelu, les ongles et/ou les muqueuses.

La partie expérimentale présentent les résultats obtenus des études *in vitro* et *in vivo* sur l'effet d'un exemple d'au moins un dérivé de carbohydrate. Des exemples de compositions utilisées selon l'invention pour améliorer la fonction barrière sont indiqués sans être limitatifs.

### PARTIE EXPERIMENTALE

### Description des figures :

**Figure 1 :** La figure 1 est un graphe illustrant l'effet du O-octanoyl-6'-maltose sur les glycosidases du stratum corneum. L'activité est exprimée en pourcentage d'activité par rapport à la valeur mesurée en absence du O-octanoyl-6'-maltose.
**Figure 2** : La figure 2 illustre l'effet à différentes concentrations sur l'activité β-glucosidase du O-octanoyl-6'-maltose, du O-octanoyl-6'-glucose, du maltose et du glucose.
**Figure 3** : La figure 3 illustre l'effet du O-octanoyl-6'-maltose sur chaque glycosidase recombinante testée (Clonezyme).

### 1. Etude d'un dérivé de carbohydrate, l' O-octanoyl-6'-maltose [α-D-glucopyrannosyl-1-4-D-glucopyrannose] sur la stimulation des activités glycosidases

### 1.1 Effet du O-octanoyl-6'-maltose sur l'activité des glycosidases du stratum corneum

Afin de déterminer la nature et l'importance de l'effet barrière d'un dérivé de carbohydrate selon l'invention, l'influence du O-octanoyl-6'-maltose sur l'activité glycosidase a été étudiée à l'aide du dosage enzymatique suivant :

Le dosage est réalisé en plaque de 96 puits. On réalise un mélange comprenant :
- 75 µl d'une solution 10 mM de substrat chromogénique spécifique couplé au para-nitro-phénol (PNP),
- 10 µl de tampon citrate 700 mM / phosphate de sodium 200 mM, pH 4,5,
- 55 µl d'une solution 0,5 M de O-octanoyl-6'-maltose[α-D-glucopyrannosyl-1-4-D-glucopyrannose],
- 40 µl de solution de glycosidases solubles du *stratum comeum.* Cette solution est obtenue par simple grattage de l'avant-bras dans un tampon citrate de sodium 70 mM, pH 4,5 contenant 1 % de Tween 20 et élimination des débris cellulaires par filtration séquentielle sur membrane de porosité de 0,45 et 0,22 µm.

Les mesures sont réalisées par quantification de la coloration jaune après une incubation à 37°C d'une durée comprise entre 1 h30 et 48 heures.

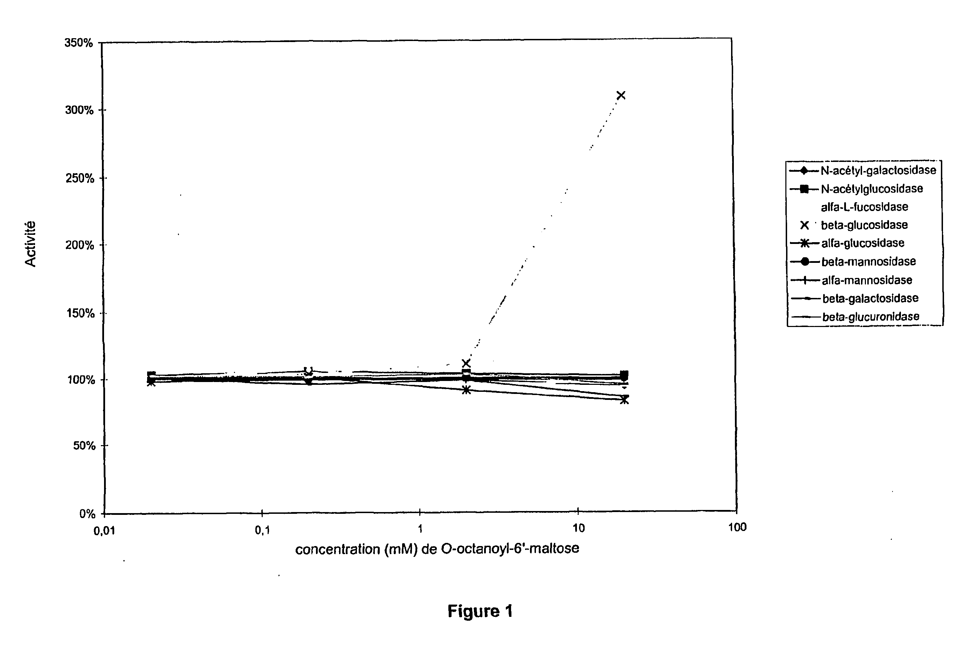

Les résultats présentés dans la figure 1 montrent que parmi les glycosidases testées, seule la β-D-glucosidase montre une cinétique influencée par le O-octanoyl-6'-maltose. Les autres glycosidases sont soit indifférentes à la présence du O-octanoyl-6'-maltose, soit très faiblement inhibées. Les β-D-glucosidases (lysosomiales) sont impliquées dans le catabolisme des glycolipides et en particulier les glucosyl-céramides en céramides. Elles sont donc spécifiquement impliquées dans la fonction barrière.

### 1.2 Concentration optimale du O-octanoyl-6'-maltose

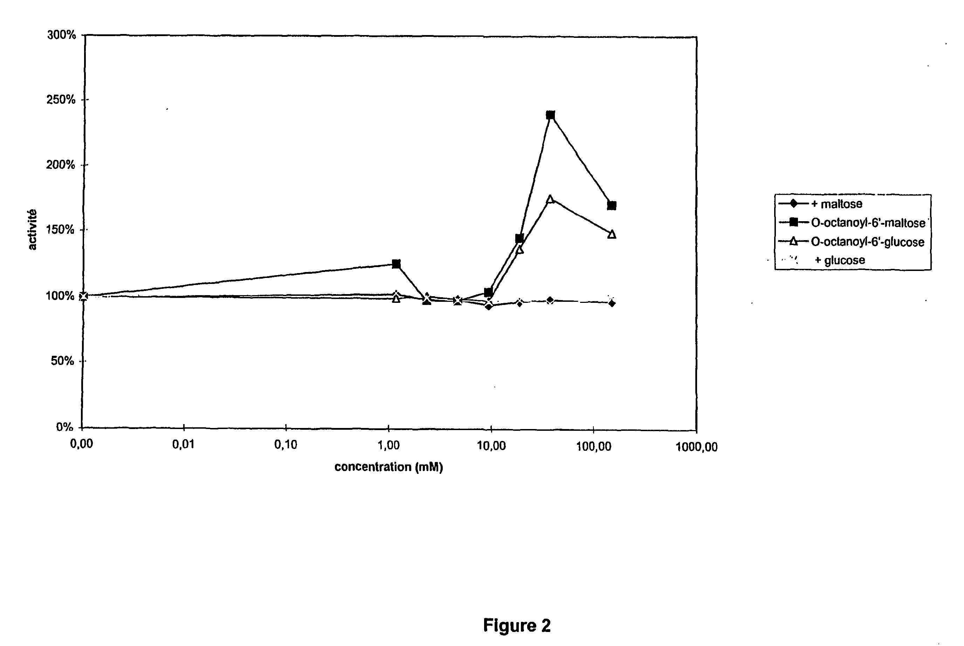

La concentration optimale de O-octanoyl-6'-maltose pour une activité β-D-glucosidase la plus élevée a été recherchée par la comparaison de concentrations croissantes de produits. Les résultats présentés dans la figure 2 montrent qu'un maximum de stimulation est observé pour des concentrations situées vers 40 mM (1%). Ce résultat montre que le O-octanoyl-6'-maltose peut être utilisé à une concentration d'environ 1 % dans une composition cosmétique destinée à stimuler l'activité β-glucosidase et par là favoriser la fonction barrière, cette concentration se situe dans une gamme cosmétologiquement acceptable. Ni le maltose seul, ni le glucose seul, ne permet de stimuler l'activité β-D-glucosidase, en revanche le O-octanoyl-6-D-glucose révèle aussi une certaine stimulation de l'activité, bien que plus faible, indiquant par là que la combinaison carbohydrate-chaine carbonée est le facteur qui agit sur l'activité glucosidase.

### 1.3 Effet du O-octanoyl-6-D-maltose sur les glycosidases thermostables recombinantes

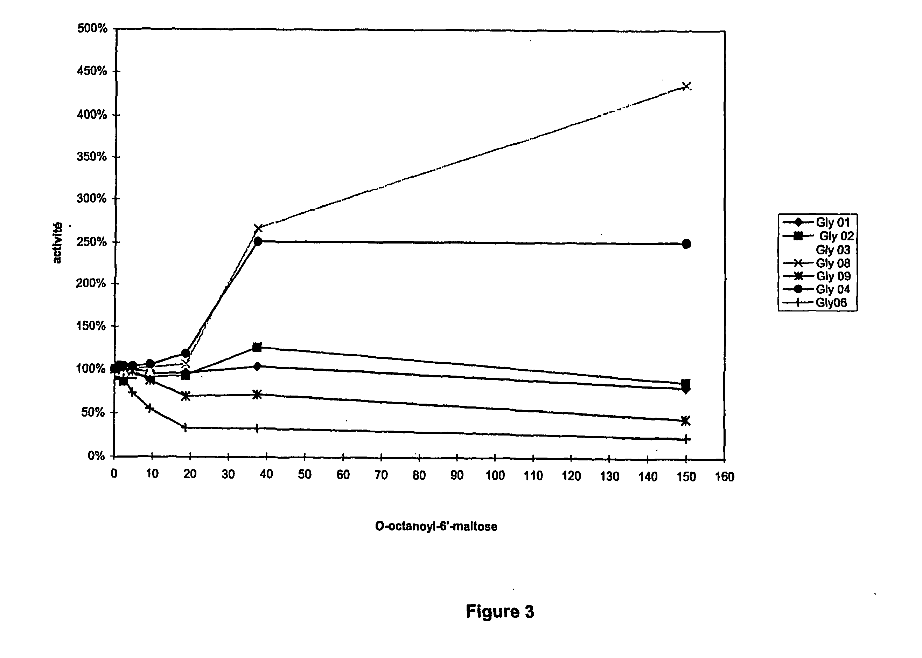

Le O-octanoyl-6'-maltose a aussi été testé pour son effet sur les glycosidases commercialisées par Clonezyme. Le tableau 1 résume les spécificités de substrats de chaque glycosidase. La figure 3 présente les résultats concernant 7 glycosidases testées en présence de concentrations croissantes en O-octanoyl-6'-maltose.

**Tableau 1**

| | GLY-01 | GLY-02 | GLY-03 | GLY-04 | GLY-05 | GLY-06 | GLY-07 | GLY-08 | GLY-09 | GLY-10 |
|---|---|---|---|---|---|---|---|---|---|---|
| β-D-cellobiose | - | ++ | ε | ε | | + | + | ε | ε | - |
| β-D-galactose | - | ++ | ε | + | + | ε | ε | + | - | - |
| α-D-glucose | - | - | - | - | - | - | - | - | - | - |
| β-D-glucose | ++ | ++ | ++ | ++ | ++ | ++ | ++ | ++ | ++ | - |
| β-N-acetyl-D-glucosaminide | - | - | - | - | - | - | - | - | - | - |
| β-D-fucose | - | ++ | ++ | ++ | ++ | ++ | ++ | + | ++ | - |
| β-L-fucose | - | - | - | - | - | - | - | - | - | - |
| β-D-glucuronide | - | - | - | - | - | - | ε | - | - | - |
| α-D-galactose | - | - | - | - | - | - | - | - | - | + |
| β-D-mannose | - | ε | - | - | - | + | + | - | + | - |
| α-D-mannose | - | - | - | - | - | - | - | - | - | - |
| β-D-xylose | ε | + | - | ε | ε | ε | ε | ε | ε | - |
| α-L-arabinofuranoside | - | - | - | - | - | - | - | - | - | - |
| α-L-arabinopyranoside | - | + | ε | ε | ε | ε | ε | ε | ε | - |
| β-D-lactose | - | + | ε | - | - | + | ε | - | - | - |
| α-L-rhamnose | - | - | - | - | - | - | - | - | - | - |
| α-D-N-acetylneuramide | - | - | - | - | - | - | - | - | - | - |
| β-D-N-acetylchitobioside | - | - | - | - | - | - | - | - | - | - |
| α-L-fucose | - | - | - | - | - | - | - | - | - | - |

| | | | | | | | | | | |
|---|---|---|---|---|---|---|---|---|---|---|
| (- : pas d'activité ; ε, +,++ : activité spécifique) | | | | | | | | | | |

Les données de la figure 3 montrent que toutes les glycosidases testées ne sont pas stimulées de la même manière en présence de O-octanoyl-6'-maltose. Un premier groupe d'enzymes est insensible. Un deuxième groupe d'enzymes est inhibé. Un troisième groupe montre une stimulation allant jusqu'à 400% de leur activité de base.

En conclusion, cette étude a permis de montrer que :
1) les dérivés carbohydrates activent spécifiquement les β-glucosidases, et en particulier, celles présentes dans le stratum corneum ;
2) cet effet est plus faible voire absent ou opposé pour les autres activités glycosidases testées, suggérant un effet stimulateur de ces produits, spécifique sur les β-glucosidases.

### 2. Effet in vivo du O-octanoyl-6'-maltose sur l'amélioration de la fonction barrière de la peau

La stimulation du O-octanoyl-6'-maltose sur l'activité β-D-glucosidase, spécifiquement, suggère un effet des dérivés de carbohydrates améliorant la fonction barrière de la peau. Une étude a été réalisée afin de vérifier *in vivo* cette hypothèse.

Le but de l'étude a été l'évaluation sur 4 semaines de l'effet du O-octanoyl-6'-maltose sur la fonction barrière.

### 2.1 Protocole expérimental

**Schéma expérimental** : L'étude a compris 70 volontaires, les sujets sont de sexe féminin de 18 à 50 ans et présentent sur les jambes une peau sèche (score > 2) et une perte insensible en eau (PIE) mesurée en g/m².h supérieure à 8.

Pour chaque individu, une jambe a été traitée et une jambe n'a pas été traitée (droite ou gauche choisie de manière aléatoire), constituant ainsi deux groupes statistiques (traité, et non traité).

La valeur moyenne de la PIE est de 10,79. La valeur moyenne du score de sécheresse est de 2,68.

Une application biquotidienne est effectuée sur la jambe à traiter suivant une randomisation droite/gauche. L'effet barrière est évalué à l'aide des paramètres PIE (perte insensible en eau) et Lag time (temps d'apparition en seconde de la rougeur due au nicotinate de méthyle).

Afin de mesurer la perte insensible en eau (PIE), le Téwamètre Courage et Khasaka est utilisé selon les recommandations du fournisseur.

Afin de réaliser la mesure du Lag time après application du nicotinate de méthyle, un laser Doppler Perimed est utilisé selon les recommandations du fournisseur.

Une analyse statistique des données est ensuite réalisée :
- Les données moyennes à 0 semaine (T0) et à 4 semaines (T4) sont comparées à l'aide d'un test t de Student pour les séries appariées.
- La comparaison des moyennes des zones traitées et des zones témoins à T4 est réalisée à l'aide d'un test t de Student pour les séries appariées.
- Les différents traitements sont comparés par une analyse de variance à un facteur (le produit). Un test de Thukey permet des comparaisons multiples 2 à 2 des moyennes (Traité contre peau nue) à T4 (4 semaines).
- Les effets observés sont quantifiés par la valeur de la moyenne des pourcentages d'évolution de la zone traitée diminuée de la moyenne des pourcentages de la zone témoin.

### 2.2 Résultats

Les valeurs moyennes et les écart-types des paramètres Lag time et PIE sont indiqués à T0 et à T4. NS, signifie un résultat négatif au test (Non Significatif), S signifie un résultat positif au test (significatif). Les traitements sont précisés dans la première colonne :
- **Véhicule**, composition comprenant uniquement le véhicule constitué d'un mélange standard de :
   Huile 12%
   Epaississant (Colomer) 0,3%
   Tensioactifs non ioniques 5% (PEG - SQ stéarate (Myrj) 2,5% et Glyceryl stérate/PEG-100 stéarate (Arlacel) 2,5%) 100 stéarate (Arlacel) 2,5%
   Eau QSP 100%
- **Peau nue,** absence de traitement ;
- **Carbohydrate,** composition comprenant 2,17% de O-octanoyl-6'-maltose[α-D-glucopyrannosyl-1-4-D-glucopyrannose] ;

Le tableau 1 ci-dessous présente l'effet des différents traitements à T0 et à T4 et la comparaison des paramètres Lag Time et PIE avant et après traitement.

**Tableau 1 : Etude en fonction du temps.**

| | **Lag Time** | | | **PIE** | | |
|---|---|---|---|---|---|---|
| | **T0** | **Signif.** | **T4** | **T0** | **Signif.** | **T4** |
| **Véhicule** | 215±85 | **S** p<0,001 | 391±102 | 7,25±1,17 | **S** p=0,015 | 6,26±1,51 |
| **Peau nue** | 230±59 | **S** p<0,001 | 374±86 | 7,23±1,55 | NS | 6,87±2,19 |
| **Carbohydrate** | 243±115 | **S** p<0,001 | 448±146 | 7,32±1,59 | **S** p=0,003 | 6,22±0,86 |
| **Peau nue** | 248±114 | **S** p=0,01 6 | 389±195 | 7,05±1,62 | NS | 7,05±1,41 |

Le tableau 2 ci-dessous est une comparaison des paramètres Lag Time et PIE après chaque traitement contre le contrôle sans traitement (peau nue).

**Tableau 2 : Etude des effets traitements à 4 semaines.**

| | **Lag Time** | **PIE** |
|---|---|---|
| **Véhicule** | 391±102 | 6,26±1,51 |
| **Significativité** | **NS** | **S** p=0,037 |
| **Peau nue** | 374±86 | 6,87±2,19 |
| **Carbohydrates** | 448±146 | 6,22±0,86 |
| **Significativité** | **NS** | **S** p=0,002 |
| **Peau nue** | 389±195 | 7,05±1,41 |

Le tableau 3 ci-dessous présente les valeurs des effets observés pour chaque traitement par rapport au contrôle sans traitement à T4.

**Tableau 3 : Comparaison des traitements**

| | **Lag Time** | **PIE** |
|---|---|---|
| | **T4_{Traité} - T4_{Témoin}** | **T4_{Traité} - T4_{Témoin}** |
| **Véhicule** | 17 ± 80 | -0,61±1,10 |
| **Carbohydrate** | 59±168 | -0,83±0,92 |

Le tableau 4 ci-dessous présente les pourcentages d'évolution des paramètres pour chaque traitement par rapport au contrôle sans traitement.

**Tableau 4 : Pourcentage d'évolution à 4 semaines**

| | **Lag Time** | **PIE** |
|---|---|---|
| **Véhicule** | 5% | - 9% |
| **Carbohydrate** | 15% | -12% |

### 2.3 Discussion des résultats

Les résultats montrent que le véhicule et le traitement « carbohydrate » permettent une diminution significative de la PIE comparativement à la peau nue (voir Tableau 2).

Il faut par ailleurs remarquer que le traitement « carbohydrate » permet une diminution significative de la PIE (-12%) (voir Tableau 4).

Ce résultat indique un effet « in vivo » de l'O-octanoyl-6'-maltose sur l'amélioration de la fonction barrière de la peau, en particulier via une diminution significative de la PIE.

### 3. Exemples de formulations

### Composition 1 : Lait pour le visage

- Huile de vaseline 7,0 g
- O-octanoyl-6'-maltose 1,0 g
- Monostéarate de glycéryle, stéarate de polyéthylène glycol(100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.

Le pH de la composition est d'environ 5.5.

### Composition 2 : Lotion

- O-octanoyl-6'-maltose 0,5 g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait

- Palmitate d'octyle 35,0 g
- Glygérine 2,0 g
- O-octanoyl-6'-maltose 0,8 g
- Polymère réticulé acrylates C10-C30/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,0 g
- O-octanoyl-6'-maltose 1,0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- O-octanoyl-6'-maltose 1,0 g
- Triéthanolamine 0,8 g
- Carbomer 0,5 g
- Conservateur qs
- Eau qsp 100 g

Le gel obtenu possède de bonnes propriétés cosmétiques.

## Revendications

1. Utilisation dans une composition cosmétique du O-octanoyl-6'-maltose pour améliorer la fonction barrière de la peau.

2. Utilisation selon la revendication 1, **caractérisée en ce que** la composition comporte en outre au moins un carbohydrate ou dérivé de carbohydrate répondant à la formule générale (I),
R-X-A (I)
dans laquelle A représente une chaîne composée de une à vingt unités carbohydrate ou dérivé de carbohydrate, comprenant chacune 3 à 6 atomes de carbone, reliées entre elles, de préférence par des ponts acétals, chacune de ces unités pouvant être éventuellement substituée, par exemple par un halogène, par une fonction amine, une fonction acide, une fonction ester, un thiol, une fonction alcoxy, une fonction thio-éther, une fonction thio-ester, une fonction amide, une fonction carbamate, une fonction urée,
R représente une chaîne alkyle ou une chaîne alcényle, comprenant de 1 à 24, préférentiellement de 4 à 24, atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate ou une fonction urée,
X représente une fonction reliant R et A,
ledit carbohydrate ou dérivé de carbohydrate ayant une activité de stimulation de l'activité β-glucosidase,

3. Utilisation selon la revendication 2, **caractérisée en ce que** X est une fonction amine, éther, amide, ester, urée, carbamate, thioester, thioéther ou sulfonamide.

4. Utilisation selon l'une des revendications 2 ou 3, **caractérisée en ce que** R comprend 4 à 16 atomes de carbone, et notamment est le radical n-butyle, n-octyle, 2-éthyl-hexyle ou n-dodécyle.

5. Utilisation selon l'une des revendications 2 à 4, **caractérisé en ce que** le carbohydrate est choisi parmi le N-butanoyl-D-glucosamine, le N-octanoyl-D-glucosamine, le N-octyloxycarbonyl-N-méthyl-D-glucamine, le N-2-éthyl-hexyloxycarbonyl-N-méthyl-D-glucamine ou le 6'-0-dodécanoyl-D-maltose.

6. Utilisation selon l'une des revendications 2 à 5, **caractérisée en ce qu'**un dérivé de carbohydrate est le O-octanoyl-6-glucose.

7. Utilisation selon l'une quelconque des revendications 1 à 6, **caractérisée en ce que** l'octanoyl 6' maltose ou son mélange avec le(s) carbohydrate(s) (I) est(sont) présent(s) en une quantité allant de 0,05% à 20% en poids par rapport au poids total de la composition.

8. Utilisation selon l'une quelconque des revendications 1 à 7, **caractérisée en ce que** l'octanoyl-6'-maltose ou son mélange avec le(s) carbohydrate(s) (I) est(sont) présent(s) en une quantité allant de 0,2% à 10% et de préférence de 0,5 à 5% en poids par rapport au poids total de la composition.

9. Utilisation selon l'une quelconque des revendications 1 à 8, **caractérisée en ce que** la composition comprend également au moins un filtre solaire complémentaire, actif dans l'UVA et/ou l'UVB, hydrophile ou lipophile, et comportant éventuellement une fonction sulfonique.

10. Utilisation selon l'une quelconque des revendications 1 à 9, **caractérisée en ce que** la composition est appropriée pour l'administration topique.

11. Procédé de traitement cosmétique de la peau destiné à améliorer la fonction barrière consistant à appliquer sur la peau une composition contenant au moins un carbohydrate telle que définie à l'une quelconque des revendications 1 à 10.

## Claims

1. Use of O-octanoyl-6'-maltose in a cosmetic composition for improving the skin barrier function.

2. Use according to claim 1, **characterized in that** the composition further comprises at least one carbohydrate or carbohydrate derivative with general formula (I):
R-X-A (I)
in which A represents a chain composed of one to twenty carbohydrate units or carbohydrate derivatives each containing 3 to 6 carbon atoms, connected together, preferably via acetal bridges, each of said units possibly being substituted, for example with a halogen, an amine function, an acid function, an ester function, a thiol, an alkoxy function, a thio-ether function, a thio-ester function, an amide function, a carbamate function or a urea function;
R represents linear or branched alkyl chain or an alkenyl chain, containing 1 to 24, preferably 4 to 24 carbon atoms, which can be interrupted by ether bridges, optionally carrying a hydroxyl function, a carboxylic acid function, an amine function, an ester function, an acyloxy function, an amide function, an ether function, a carbamate function or a urea function,
X represents a function connecting R and A;
said carbohydrate or carbohydrate derivative having a stimulant effect on the β-glucosidase activity.

3. Use according to claim 2, **characterized in that** X is an amine, ether, amide, ester, urea, carbamate, thioester, thioether or sulphonamide function.

4. Use according to any of claims 2 or 3, wherein R comprises 4 to 16 carbon atoms, and is namely an n-butyl, n-octyl, 2-ethylhexyl or n-dodecyl radical.

5. Use according to any of claims 2 to 4, **characterized in that** the carbohydrate is chosen from N-butanoyl-D-glucosamine, N-octanoyl-D-glucosamine, N-octyloxycarbonyl-N-methyl-D-glucamine, N-2-ethylhexyloxycarbonyl-N-methyl-D-glucamine, or 6'-O-dodecanoyl-D-maltose.

6. Use according to claim 1, **characterized in that** the carbohydrate derivative is O-octanoyl-6-glucose.

7. Use according to any one of claims 1 to 6, **characterized in that** the O-octanoyl-6'-maltose or mixture thereof with carbohydrate(s) according to (I) is present in a quantity of 0.05% to 20% by weight with respect to the total composition weight.

8. Use according to any one of claims 1 to 7, **characterized in that** the O-octanoyl-6'-maltose or mixture thereof with carbohydrate(s) according to (I) is present in a quantity of 0.2% to 10%, preferably 0.5% to 5% by weight with respect to the total composition weight.

9. Use according to any one of claims 1 to 8, **characterized in that** the composition also comprises at least one complementary hydrophilic or lipophilic sunscreen which is active in the UVA and/or UVB region, optionally comprising a sulphonic function.

10. Use according to any of claim 1 to 9, **characterized in that** the composition is suitable for topical administration.

11. A method for cosmetic treatment of the skin to improve the barrier function, consisting of applying a composition containing at least one carbohydrate as defined in any of claims 1 to 10 to the skin.

## Patentansprüche

1. Verwendung von O-Octanoyl-6'-maltose in einer kosmetischen Zusammensetzung zur Verbesserung der Barrierefunktion der Haut.

2. Verwendung gemäß Anspruch 1, **dadurch gekennzeichnet, dass** die Zusammensetzung außerdem mindestens ein Kohlenhydrat oder Kohlenhydrat-Derivat der allgemeinen Formel (I) aufweist,
R-X-A (I)
worin A eine Kette darstellt, die aus einer bis zwanzig Kohlenhydrat- oder Kohlenhydrat-Derivat-Einheiten, die jeweils 3 bis 6 Kohlenstoffatome umfassen, zusammengesetzt ist, die miteinander verbunden sind, vorzugsweise über Acetalbrücken, wobei jede dieser Einheiten gegebenenfalls substituiert sein kann, beispielsweise mit einem Halogen, mit einer Aminogruppe, einer Säuregruppe, einer Estergruppe, einem Thiol, einer Alkoxygruppe, einer Thioethergruppe, einer Thioestergruppe, einer Amidgruppe, einer Carbamatgruppe, einer Harnstoffgruppe,
R eine Alkylkette oder eine Alkenylkette darstellt, die 1 bis 24, vorzugsweise 4 bis 24 Kohlenstoffatome umfasst, verzweigt oder geradkettig, die durch Etherbrücken unterbrochen sein kann und die gegebenenfalls eine Hydroxylgruppe, eine Carbonsäuregruppe, eine Aminogruppe, eine Estergruppe, eine Acyloxygruppe, eine Amidgruppe, eine Ethergruppe, eine Carbamatgruppe oder eine Harnstoffgruppe aufweist,
X eine Gruppe darstellt, die R und A verbindet,
wobei das Kohlenhydrat oder Kohlenhydrat-Derivat eine Wirkung besitzt, die β-Glucosidaseaktivität zu stimulieren.

3. Verwendung gemäß Anspruch 2, **dadurch gekennzeichnet, dass** X eine Amino-, Ether-, Amid-, Ester-, Harnstoff-, Carbamat-, Thioester-, Thioether- oder Sulfonamidgruppe ist.

4. Verwendung gemäß einem der Ansprüche 2 oder 3, **dadurch gekennzeichnet, dass** R 4 bis 16 Kohlenstoffatome umfasst und insbesondere ein n-Butyl-, n-Octyl-, 2-Ethylhexyl- oder n-Dodecyl-Radikal ist.

5. Verwendung gemäß einem der Ansprüche 2 bis 4, **dadurch gekennzeichnet, dass** das Kohlenhydrat ausgewählt ist aus N-Butanoyl-D-glucosamin, N-Octanoyl-D-glucosamin, N-Octyloxycarbonyl-N-methyl-D-glucamin, N-2-Ethylhexyloxycarbonyl-N-methyl-D-glucamin oder 6'-O-Dodecanoyl-D-maltose.

6. Verwendung gemäß einem der Ansprüche 2 bis 5, **dadurch gekennzeichnet, dass** ein Kohlenhydrat-Derivat O-Octanoyl-6-glucose ist.

7. Verwendung gemäß einem der Ansprüche 1 bis 6, **dadurch gekennzeichnet, dass** Octanoyl-6'-maltose oder deren Gemisch mit Kohlenhydrat(en) (I) in einer Menge vorliegt, die von 0,05 Gew.-% bis 20 Gew.-%, bezogen auf das Gesamtgewicht der Zusammensetzung, reicht.

8. Verwendung gemäß einem der Ansprüche 1 bis 7, **dadurch gekennzeichnet, dass** Octanoyl-6'-maltose oder deren Gemisch mit Kohlenhydrat(en) (I) in einer Menge vorliegt, die von 0,2 Gew.-% bis 10 Gew.-%, und vorzugsweise von 0,5 bis 5 Gew.-%, bezogen auf das Gesamtgewicht der Zusammensetzung, reicht.

9. Verwendung gemäß einem der Ansprüche 1 bis 8, **dadurch gekennzeichnet, dass** die Zusammensetzung auch mindestens einen hydrophilen oder lipophilen, komplementären Sonnenfilter umfasst, der im UVA- und/oder UVB-Bereich aktiv ist und gegebenenfalls eine Sulfonsäuregruppe aufweist.

10. Verwendung gemäß einem der Ansprüche 1 bis 9, **dadurch gekennzeichnet, dass** die Zusammensetzung zur topischen Verabreichung geeignet ist.

11. Verfahren der kosmetischen Behandlung der Haut, die der Verbesserung der Barrierefunktion dient, bestehend aus dem Auftragen einer Zusammensetzung, die mindestens ein Kohlenhydrat enthält, wie es durch einen der Ansprüche 1 bis 10 definiert ist, auf die Haut.
